# EUROPEAN PATENT SPECIFICATION

(11) **EP 3 858 303 B1**
(45) Date of publication and mention of the grant of the patent: **19.11.2025**
(21) Application number: 20020644.9
(22) Date of filing: 23.12.2020
(51) Int. Cl.: A61F 9/00, B65D 47/18

(54) **A DISPENSER FOR DISPENSING LIQUIDS**
AUSGABEEINRICHTUNG FÜR FLÜSSIGKEITEN
DISPOSITIF DE DISTRIBUTION DE LIQUIDES

(30) Priority: 03.02.2020 PL 43282820
(43) Date of publication of application: 04.08.2021
(73) Proprietor: Gerresheimer Boleslawiec Spolka Akcyjna, 59-700 Boleslawiec (PL)
(72) Inventor: SLIWA, Mateusz, 59-600 Lwówek Slaski (PL)
(74) Representative: Sleczka, Joanna

(56) References cited:
- EP-A1- 3 059 181
- FR-A1- 2 934 569
- FR-A1- 2 941 682
- US-A- 5 845 814
- US-A- 5 992 701
- US-A1- 2005 173 456
- US-A1- 2011 297 703

## Description

The object of the invention is a dispenser for dispensing liquids, in particular ophthalmic liquids, where the medicine is usually dispensed as individual drops.

There is a known dispenser for dispensing liquids, in particular ophthalmic liquids, presented in the description of application of the invention P-426592. A dispenser for dispensing liquids, mounted on the neck of a reservoir comprising a flange (26) connected to a bushing (24) and with a domed tip (36) is known from the document US 2005/173456 A1.

This dispenser is mounted on the neck of a reservoir, in particular a bottle, and it is made of a flange fitted onto the neck of the reservoir and connected to the flange of a bushing entering the neck of the reservoir, and also connected to the flange of a portion closing the neck of the reservoir, which in its central zone has a cylindrical tongue entering the neck of the reservoir, while a narrow opening is formed in the axis of the tongue. Dispensers according to this application are characterised in that a cylindrical recess is formed in the axis of the tongue, in which an inset is placed, made of foamed plastic, preferably polyethylene or polypropylene. The inset of foamed plastic has pores 7 - 30 micrometres in size. The recess for the inset has the following dimensions: diameter 1.5 - 2.5 mm, length 2.5 - 4.5 mm.

The dispenser for dispensing liquids, in particular ophthalmic liquids, according to the invention, is mounted on the neck of a reservoir, in particular a bottle; it is made of a flange connected to a bushing and with a domed tip situated on the opposite side of the bushing, the flange being fitted onto the neck of the reservoir and the bushing entering the neck of the reservoir, while a dispensing domed tip comprising a dispensing opening protrudes outside the reservoir. The dispenser is characterised in that inside the bushing and the domed tip there is a conical element placed therein, blocking the flow of liquids, the opening in the domed tip having an inner flange. The conical blocking element has a flange at the base of the cone. The flange situated in the dispensing domed tip is ended from the inside with a fin directed towards the conical element blocking the flow of liquids. The fin directed towards the conical element blocking the flow of liquids has a local gap with the following dimensions: A from 0.3 to 0.6 mm and B from 0.3 to 0.6 mm. In the place of connection to the domed tip, the bushing has a flange constituting a stop for the conical element, wherein in the flange there is a cut-out with the following dimensions: C from 0.3 to 0.6 mm, D from 0.3 to 0.6 mm and E from 0.3 to 0.6 mm

The dispenser for dispensing liquids according to the invention is characterised by its extremely simple structure, and at the same time good functionality, since it allows the dispensing of individual drops of liquids without any problems. This has been achieved due to the use of two flanges disposed separately and the inside placement of a conical element blocking the flow of liquids, which constitutes an obstruction for the free outflow of liquids, and at the same time it allows the outflow of a very small amount of a liquid in the form of a drop. At the same time, this element does not require such precise construction as elements in other dispensers. Moreover, it allows the free flow of air after the action of dispensing a liquid. The design of the dispenser allows avoiding high costs resulting from complicated dispensing structures and complicated structures introducing air into the reservoir.

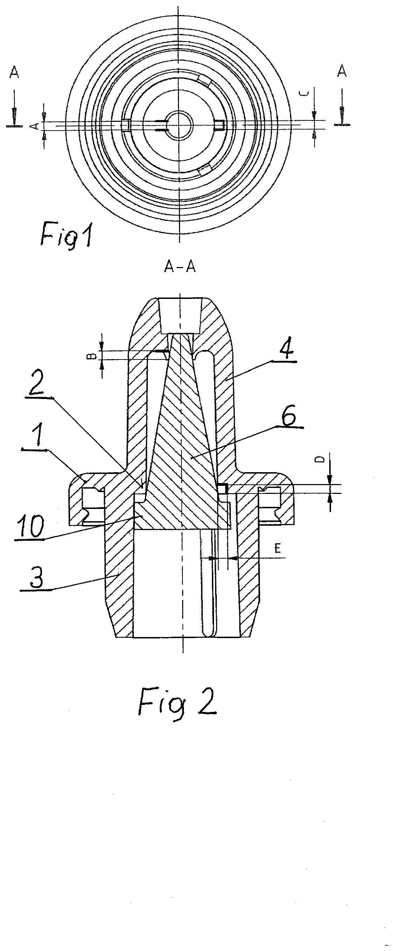

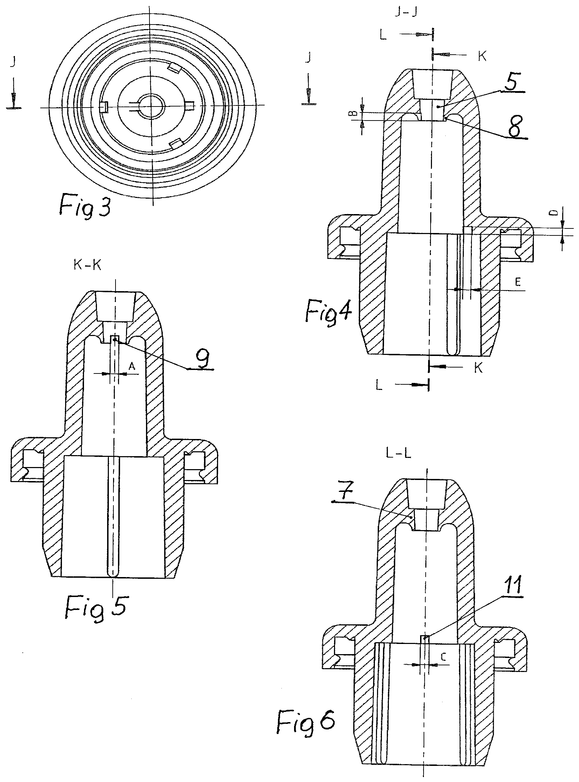

The dispenser for dispensing liquids according to the invention is described more closely in an embodiment and in the drawing, in which fig. 1 presents the dispenser in a top view, fig. 2 presents the dispenser in a lengthwise section along the line A-A, fig. 3 is a view of the dispenser without the conical element blocking the flow of liquids, fig. 4 constitutes a cross-section of the dispenser of fig. 3 along the line J-J, fig. 5 is a cross-section of the dispenser of fig. 3 along the line K-K, while fig. 6 is a cross-section of the dispenser of fig. 3 along the line L-L.

As presented in fig. 1 to fig. 6, the dispenser for dispensing ophthalmic liquids according to the invention is made of a flange 1 connected to a bushing 3 and to a domed tip 4 situated on the opposite side of the bushing 3. The flange 1 is fitted onto the neck of a reservoir. The flange 1 is connected to the flange 2 of the bushing 3, which is placed in the neck of the reservoir. The domed tip 4 comprises a dispensing opening 5 inside it. The conical element 6 blocking the flow of liquids is placed inside the bushing 3 and the dispensing domed tip 4. The opening 5 in the dispensing domed tip 4 has an inner flange 7. The flange 7 situated in the dispensing domed tip 4 is ended from the inside with a fin 8 directed towards the conical element 6 blocking the flow of liquids. The fin 8 has a local gap 9, whose dimension A amounts to 0.4 mm, while its dimension B amounts to 0.3 mm. At the base of the cone, the conical element 6 has a flange 10, which during dispensing leans against the flange 2 of the bushing 3. In the flange 2 there is a cut-out with the following dimensions: C=0.4 mm, D=0.4 mm and E=0.3 mm. The dispensed medicine seeps through this cut-out.

The use of the dispenser proceeds as follows. The dispenser is fitted onto a reservoir, which is not shown in the drawing, and in particular onto its neck. The reservoir is filled with a proper liquid intended to cure the eye to which the medicine is to be delivered in the form of a single drop. To this end, the reservoir with the fitted dispenser is turned upside down. The liquid contained in the reservoir pushes against the flange 10 of the conical element 6, due to which it closes the outflow of the liquid from the reservoir and thus from the dispenser. The liquid can only seep through the cut-out between the flange 10 and the flange 2 and through the local gap 9. This means that the outflow of the curing liquid from the reservoir is obstructed in two places, due to which it is much easier to achieve a single drop, and also the construction of individual elements of the dispenser does not require the precision of previous solutions.

## Claims

1. A dispenser for dispensing liquids, in particular ophthalmic liquids, mounted on the neck of a reservoir, in particular a bottle, made of a flange connected to a bushing and with a domed tip situated on the opposite side of the bushing, the flange being fitted onto the neck of the reservoir and the bushing entering the neck of the reservoir, while a dispensing domed tip comprising a dispensing opening protrudes outside the reservoir, **characterised in that** inside the bushing (3) and the domed tip (4) there is a conical element (6) placed therein, blocking the flow of liquids, and the conical element (6) has a flange (10) at the base of the cone, wherein the opening (5) in the domed tip (4) having an inner flange (7), which is ended from the inside with a fin (8) directed towards the conical element (6) blocking the flow of liquids, and the fin (8) has a local gap (9), whereas in the place of connection to the domed tip (4), the bushing (3) has a flange (2) constituting a stop for the conical element (6), wherein in the flange (2) there is a cut-out (11).

2. The dispenser according to claim 1, **characterised in that** the local gap (9) has the following dimensions: A from 0.3 to 0.6 mm and B from 0.3 to 0.6 mm, whereas the cut-out (11) has the following dimensions: C from 0.3 to 0.6 mm, D from 0.3 to 0.6 mm and E from 0.3 to 0.6 mm.

## Patentansprüche

1. Ein Spender zum Ausgeben von Flüssigkeiten, insbesondere Augenflüssigkeiten, der am Hals eines Behälters, insbesondere einer Flasche, angebracht ist und aus einem mit einer Buchse verbundenen Flansch und einer gewölbten Spitze, die sich auf der gegenüberliegenden Seite der Buchse befindet, besteht, wobei der Flansch auf den Hals des Behälters aufgesetzt ist und die Buchse in den Hals des Behälters hineinragt, während eine gewölbte Ausgabespitze mit einer Ausgabeöffnung aus dem Behälter herausragt, **dadurch gekennzeichnet, dass** sich innerhalb der Buchse (3) und der gewölbten Spitze (4) ein konisches Element (6) befindet, das den Flüssigkeitsfluss blockiert, und das konische Element (6) einen Flansch (10) an der Basis des Konus aufweist, wobei die Öffnung (5) in der gewölbten Spitze (4) einen Innenflansch (7) aufweist, der von innen mit einem Grat (8) endet, der zum konischen Element (6) hin ausgerichtet ist, das den Flüssigkeitsfluss blockiert, und der Grat (8) einen lokalen Spalt (9) aufweist, während die Buchse (3) an der Verbindungsstelle mit der gewölbten Spitze (4) einen Flansch (2) aufweist, der einen Anschlag für das konische Element (6) bildet, wobei sich im Flansch (2) eine Aussparung (11) vorhanden ist.

2. Der Spender gemäß Anspruch 1, **dadurch gekennzeichnet, dass** der lokale Spalt (9) die folgenden Abmessungen aufweist: A von 0,3 bis 0,6 mm und B von 0,3 bis 0,6 mm, während die Aussparung (11) die folgenden Abmessungen aufweist: C von 0,3 bis 0,6 mm, D von 0,3 bis 0,6 mm und E von 0,3 bis 0,6 mm.

## Revendications

1. Distributeur pour distribuer des liquides, en particulier des liquides ophtalmiques, monté sur le col d'un réservoir, en particulier d'un flacon, fait d'une bride reliée à une douille et d'un embout bombé situé du côté opposé à la douille, la bride étant ajustée sur le col du réservoir et la douille pénétrant dans le col du réservoir, tandis qu'un embout bombé de distribution comprenant une ouverture de distribution fait saillie à l'extérieur du réservoir, **caractérisé en ce qu'**à l'intérieur de la douille (3) et de l'embout bombé (4) se trouve un élément conique (6) placé là, bloquant l'écoulement de liquides, et l'élément conique (6) présente une bride (10) au niveau de la base du cône, où l'ouverture (5) dans l'embout bombé (4) présentant une bride intérieure (7), qui se termine de l'intérieur par une ailette (8) dirigée vers l'élément conique (6) bloquant l'écoulement de liquides, et l'ailette (8) présente un espace local (9), tandis qu'à l'endroit de liaison avec l'embout bombé (4), la douille (3) présente une bride (2) constituant une butée pour l'élément conique (6), où il existe une découpe (11) dans la bride (2).

2. Distributeur selon la revendication 1, **caractérisé en ce que** l'espace local (9) présente les dimensions suivantes : A de 0,3 à 0,6 mm et B de 0,3 à 0,6 mm, tandis que la découpe (11) présente les dimensions suivantes : C de 0,3 à 0,6 mm, D de 0,3 à 0,6 mm et E de 0,3 à 0,6 mm.
